# EUROPEAN PATENT APPLICATION

(11) **EP 4 343 687 A1**
(43) Date of publication of application: **27.03.2024**
(21) Application number: 22197625.1
(22) Date of filing: 26.09.2022
(51) Int. Cl.: G06T 7/00, A61B 6/00, G06T 7/11, G06V 10/00

(54) **IMAGE SEGMENTATION WITH IMPROVED EXPERT GUIDANCE**

(71) Applicant: Koninklijke Philips N.V., 5656 AG Eindhoven (NL)
(72) Inventor: KUMAR, Vinod, Eindhoven (NL); RAO KUNDETI, Srinivasa, Eindhoven (NL); SHIVASHANKAR, Bharath, 5656AG Eindhoven (NL); SISODIA, Rajendra Singh, 5656AG Eindhoven (NL)
(74) Representative: Philips Intellectual Property & Standards

(57) **Abstract**

Some embodiments relate to machine learnable image segmentation. An image segmentation model may be applied to a set of input images thus obtaining a corresponding set of segmentation images. Corrected segmentation images obtained from expert judgement may be used to train a further image segmentation model predicting an expected error image.

## Description

### TECHNICAL FIELD

The presently disclosed subject matter relates to a method for a machine learnable image segmentation model, a device for a machine learnable image segmentation model, a computer readable medium.

### BACKGROUND

Medical image analysis, such as segmentation and classification for diagnosis has achieved a remarkable performance. For example, deep learning based models can produce medical image segmentation that are of high quality. There remain however images on which a given image segmentation model may give the wrong answer. Experiments on image classification have shown that a trained model sometimes fails on specific instances, despite being very confident in its prediction

As pointed out in the paper "Aleatoric and Epistemic Uncertainty in Machine Learning: An Introduction to Concepts and Methods", by Eyke Hüllermeier and Willem Waegeman, two sources of uncertainty can be identified: aleatoric and epistemic. According to these authors, aleatoric (aka statistical) uncertainty refers to the notion of randomness, that is, the variability in the outcome of an experiment which is due to inherently random effects. Epistemic (aka systematic) uncertainty refers to uncertainty caused by a lack of knowledge. Epistemic uncertainty can in principle be reduced using additional information.

Indeed, an important aspect in the success of these new image segmentation models is the quality of the ground truth training data on which the image models are trained. For best performance, human domain expert evaluate large numbers of image to improve training and refining of the model on.

It would be advantageous to obtain more data of high quality, so reduce epistemic uncertainty in image segmentation models.

### SUMMARY

A first possible workflow to obtain better training data is to use a partially trained image segmentation model. The partially trained image segmentation model may be applied to a set of images thus obtaining a corresponding set of segmentation images. A pool of expert can now correct the segmentation images where needed instead of having to fully segment by hand. The corrected segmentation images that are so obtained can then be used to train a better model or the further train the existing model.

This workflow is more efficient as the exerts do not have to segment those parts of the images where the model is already correct. As the model gets better, the model will be correct more often than not, so that the work of the expert is significantly reduced.

A drawback of this first workflow is that experts need to carefully evaluate a segmentation proposed by the model for errors. In a second workflow, the (partially trained) image segmentation model computes not only a segmentation image, but also an uncertainty image for an input image indicating a confidence of the image segmentation model for different parts of the segmentation image. Methods to compute such uncertainty images, are known per se; some of which are cited herein. In the second workflow, the expert is confronted with a segmentation image produced by the image segmentation model, as well as with the corresponding uncertainty image. The expert is now guided to those parts of the image that are most likely to be wrong, and can thus evaluate the segmentation proposed by the model faster. As in the first workflow, the expert will correct the segmentation if needed, e.g., for training purposes.

Although, the second workflow produces high quality training data in less time, the inventors found that the second workflow is still not optimal. A problem that they observed, is that uncertainty images tend to mark large parts of an image as uncertain, even if such parts of the image will in fact not be corrected. This negates the advantage of uncertainty images in focusing attention of the expert on the part of image needing verification. Another problem is that edges of objects in an uncertainty image tend to have a ribbon of uncertainty around them. A possible reason for this ribbon is that the exact edge of an object is often not clear and can be labeled differently by various experts in the various images. Although, the uncertainty image correctly indicates that the exact boundary of the image is uncertain, the boundary is nevertheless unlikely to be corrected. The expert evaluating the image likely finds the position of an object boundary to be satisfactory, or he/she might not have a better judgement on such boundaries either.

An embodiment proposes to train a second image segmentation model in addition to the first image segmentation model that learns the desired segmentation. The second image segmentation model is trained on the corrections that experts make to the segmentations produced by the first image segmentation model. In an improved workflow, the expert may be confronted with a segmentation image produced by the first image segmentation model, as well as with a corresponding expected error image computed by the second image segmentation model.

Such a second image segmentation model has advantages over the use of uncertainty images as discussed for the second workflow. Expected error images tend to mark less of the image as possibly faulty then an uncertainty image would. The expected error image works better in focusing the attention of the expert to regions of the segmentation that might have genuine error.

Expected error images furthermore suffer less from the uncertainty ribbons, or halos, that can appear around objects. As the exact boundary of an object tends not to be corrected by experts, that is, tends not to need such correction, the second image segmentation model will correctly learn that such boundaries will probably not be shifted. This also helps in focusing the attention of the expert to those parts that mostly likely need review. Both aspects thus contribute to reducing the review time of the model's predicted segmentations.

In a third workflow, a further image segmentation model predicting an expected error image is used by having it predict an expected error image for an input image, and showing an expert both a segmentation image produced by the image segmentation model and the expected error image. The expected error image will focus attention on parts that are most likely to need correction. Accordingly, the expert can correct the segmentation quicker. This is an improved workflow for obtaining image segmentations. In particular for medical image segmentation, time to segment and label images reduces compared to the first and second workflow indicated above. The corrected segmentation may be used for a clinical purpose, e.g., diagnosis, evaluation, planning of treatment, and so on. A further image segmentation model predicting an expected error image may be used by having it predict an expected error image for an input image, and showing an expert both a segmentation image produced by the image segmentation model and the expected error image. The expected error image may be used to train the image segmentation model or to train the further image segmentation model.

For example, in an embodiment, an image segmentation model is configured to receive an input image and to produce as output a segmentation image classifying parts of the input image. A further image segmentation model predicting an expected error image for an input image may be obtained by training an image segmentation model on error images that indicate locations where a segmentation image was corrected by expert judgement. This approach works well for a medical segmentation model, but can be applied in other fields as well.

A knowledge graph is an advantageous data structure to keep track of the various images and other data occurring in embodiments, is

In an embodiment, an associated uncertainty model is configured to produce an uncertainty image for an input image indicating a confidence of the image segmentation model for different parts of the segmentation image. Such uncertainty images can be obtained without access to a trained further image model. It was found advantageous to provide an uncertainty image as an input to the further image model.

An embodiment of a method may be implemented on a computer as a computer implemented method, or in dedicated hardware, or in a combination of both. Executable code for an embodiment of the method may be stored on a computer program product. Examples of computer program products include memory devices, optical storage devices, integrated circuits, servers, online software, etc. Preferably, the computer program product comprises non-transitory program code stored on a computer readable medium for performing an embodiment of the method when said program product is executed on a computer.

In an embodiment, the computer program comprises computer program code adapted to perform all or part of the steps of an embodiment of the method when the computer program is run on a computer. Preferably, the computer program is embodied on a computer readable medium.

Another aspect of the presently disclosed subject matter is a method of making the computer program available for downloading.

### BRIEF DESCRIPTION OF DRAWINGS

Further details, aspects, and embodiments will be described, by way of example, with reference to the drawings. Elements in the Figures are illustrated for simplicity and clarity and have not necessarily been drawn to scale. In the Figures, elements which correspond to elements already described may have the same reference numerals. In the drawings,
Figure 1 schematically shows an example of an embodiment of an image segmentation device,
Figure 2a schematically shows an example of an embodiment of an image segmentation system,
Figure 2b schematically shows an example of an embodiment of an image segmentation system,
Figure 2c schematically shows an example of an embodiment of an image segmentation system,
Figure 2d schematically shows an example of an embodiment of a data structure,
Figure 2e schematically shows an example of an embodiment of a knowledge graph,
Figure 3a shows an example of an embodiment of a medical image,
Figure 3b schematically shows an example of an embodiment of a medical image,
Figure 3c schematically shows an example of an embodiment of a segmentation image,
Figure 3d schematically shows an example of an embodiment of an uncertainty image,
Figure 3e schematically shows an example of an embodiment of a corrected segmentation image,
Figure 3f schematically shows an example of an embodiment of an error image,
Figure 3g schematically shows an example of an embodiment of an expected error image
Figure 4 schematically shows an example of an embodiment of an image segmentation method,
Figure 5 schematically shows an example of an embodiment of an image segmentation method,
Figure 6a schematically shows a computer readable medium having a writable part comprising a computer program according to an embodiment,
Figure 6b schematically shows a representation of a processor system according to an embodiment.

### Reference signs list

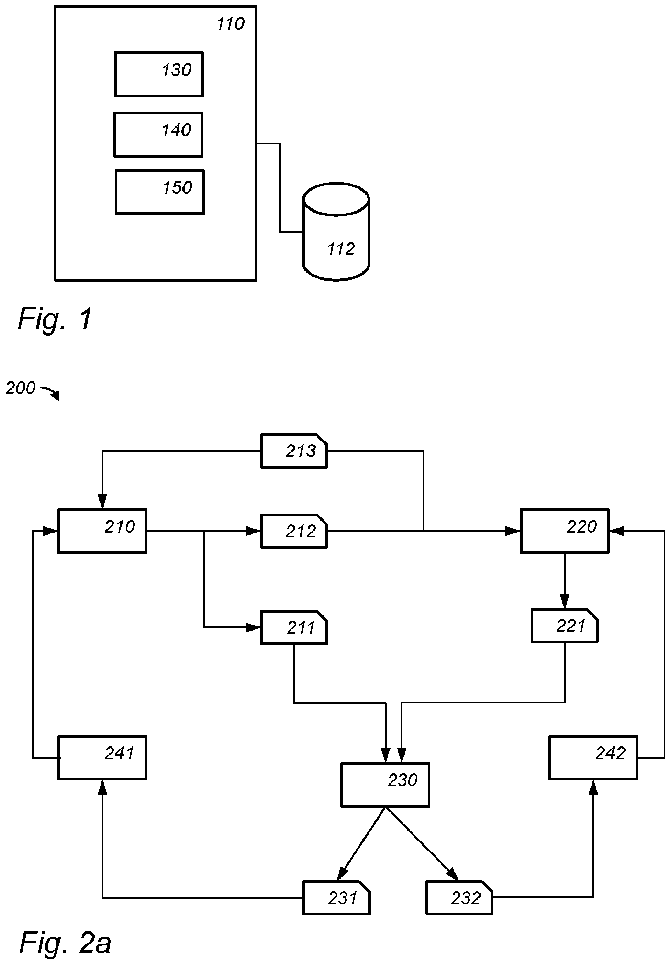

The following list of reference signs refers to Figures 1, 2a-2e, 3a-3g, 6a and 6b and is provided for facilitating the interpretation of the drawings and shall not be construed as limiting the claims.
- 110: an image segmentation device
- 112: an image database
- 130: a processor system
- 140: storage
- 150: communication interface
- 200: an image segmentation system
- 210: an image segmentation model
- 211: a segmentation image
- 212: an uncertainty image
- 213: an input image
- 215: an associated uncertainty model
- 220: a further image segmentation model
- 221: an expected error image
- 230: an expert judgement interface
- 231: a corrected segmentation image
- 232: an error image
- 233: a further corrected segmentation image
- 241: a first model trainer
- 242: a second model trainer
- 250: an expert selection unit
- 260: a knowledge graph
- 261-263: additional data
- 265: a data structure
- 300: a medical image
- 301: a schematic rendering of image 300
- 310: a segmentation image
- 320: an uncertainty image
- 321: a first region
- 322: a second region
- 330: a corrected segmentation image,
- 331: a corrected part
- 340: an error image,
- 341: a corrected part
- 350: an expected error image
- 1000,1001: a computer readable medium
- 1010: a writable part
- 1020: a computer program
- 1110: integrated circuit(s)
- 1120: a processing unit
- 1122: a memory
- 1124: a dedicated integrated circuit
- 1126: a communication element
- 1130: an interconnect
- 1140: a processor system

### DESCRIPTION OF EMBODIMENTS

While the presently disclosed subject matter is susceptible of embodiment in many different forms, there are shown in the drawings and will herein be described in detail one or more specific embodiments, with the understanding that the present disclosure is to be considered as exemplary of the principles of the presently disclosed subject matter and not intended to limit it to the specific embodiments shown and described.

In the following, for the sake of understanding, elements of embodiments are described in operation. However, it will be apparent that the respective elements are arranged to perform the functions being described as performed by them. Further, the subject matter that is presently disclosed is not limited to the embodiments only, but also includes every other combination of features described herein or recited in mutually different dependent claims.

Image segmentation model's success is based in part on advanced segmentation algorithms, e.g., based on deep convolutional neural networks, and in part on accurate training data. Advanced segmentation models, especially medical image segmentation, still requires large, representative, and high-quality annotated datasets. Obtaining a perfect training dataset is rare though, particularly in the field of medical imaging, where data and annotations are expensive to acquire. Automated AI Medical image segmentation performed with partially trained algorithms and human-assisted correction with editing tools in a radiology workflow helps to improve the training dataset quality. Such a workflow reduces the need for radiologist second reads and/or human supervision, and increases productivity.

Medical images like MRI, CT, etc., are primary modes of diagnosis for a variety of medical conditions from tumor to ligament tear. Medical Image labelling is not only a key requirement for supervised Machine Learning and Deep Learning Models but is also used for clinical purposes.

For example, one of the challenges concerns detecting aleatoric and epistemic uncertainty in machine learning, non-availability of representative data, generalization failure of labelling models, especially in a multi-site environment, non-availability of expert radiologists; intra and inter annotator variance due to different skill level of radiologists. Labor-intensive corrections may be required from the radiologist for automatically predicted segmentation masks from the AI model.

Segmentation uncertainty may originate from different source types including physical device issues, annotation tool issues, hidden features in the model or wrong decision boundaries. These uncertainties can be broadly classified into aleatoric and epistemic uncertainty. Aleatoric uncertainty relates to an objective or physical concept of uncertainty, which is intrinsic to the data. The source of aleatoric uncertainty is often unclear. Epistemic uncertainty relates to subjective or personal concept of uncertainty. It occurs due to lack of knowledge or ignoring certain features during data generation process or training the model.

An improved approach for improving AI image segmentation model performance is described herein in various embodiments. For example, an embodiment may use an uncertainty predictor used in a smart annotation workflow. A knowledge graph may advantageously be used to organize data in the system.

In a conventional smart labelling scenario, an image segmentation model may generate an approximate segmentation image, also known as a segmentation image. An expert, e.g., a radiologist, is then provided with editing tools to correct the auto segmented area(s).

A smart labelling scenario according to an embodiment, an image segmentation model may generate an approximate segmentation image. An expert is shown the segmented image and an expected error image, obtained from a model trained on previous corrections. The expert is provided with editing tools to correct the auto segmented area(s); his focus is guided by the expected error image to reduce review time.

In an embodiment, a model is trained to learn the uncertainty corrections from human annotators and model them. For example, such data may be stored in a flexible data structure such as a knowledge graph. A neural network trained on correction can be used to predict uncertain regions in the image. These uncertain images are later used to correct or evaluate the image segmentation model and/or to improve the productivity of the expert annotator. Reducing expert assistance with smart annotations that autofill with high certainty reduce radiologist second reads and/or human supervision and increases productivity.

Figure 1 schematically shows an example of an embodiment of an image segmentation device 110. Image segmentation device 110 is configured for a machine learnable image segmentation model. For example, the device 110 may be used to apply the image segmentation model to one or more input images thus obtaining corresponding segmentation images. Such a device is useful in a number of fields, in particular in the medical field, e.g., when applied to medical images. Other fields include, e.g.: manufacturing, where image segmentation may be used, say, to detect faults in manufactured products; assisted or autonomous driving where image segmentation may be used, say, to detect objects in street images.

Device 110 may be configured for obtaining a set of corrected segmentation images correcting the set of segmentation images from expert judgement and a corresponding set of error images, an error image indicating a location where a segmentation image is corrected, and training a further image segmentation model predicting an expected error image 221 for an input image. Such a trained further image segmentation model is advantageous as it gives a better indication where expert review is useful. Embodiment can be applied to image segmentation models in various fields;
medical segmentation models being a good example.

Device 110 may be configured for training the further image segmentation model, but this is not necessary. In a useful embodiment, device 110 is instead (or in addition) configured to apply a further image segmentation model that is already trained, e.g., on a different device. For example, a pretrained error prediction model may be obtained from a third party, as a useful addition to a semiautonomous segmentation workflow. The corrected segmentations that an expert produces in a semiautonomous segmentation workflow could be advantageously used in further training of both the image segmentation model and of the further image segmentation model, but this is not necessary.

Image segmentation device 110 may comprise a processor system 130, a storage 140, and a communication interface 150. Storage 140 may be, e.g., electronic storage, magnetic storage, etc. The storage may comprise local storage, e.g., a local hard drive or electronic memory. Storage 140 may comprise non-local storage, e.g., cloud storage. In the latter case, storage 140 may comprise a storage interface to the non-local storage. Storage may comprise multiple discrete sub-storages together making up storage 140. Storage may comprise a volatile writable part, say a RAM, a non-volatile writable part, e.g., Flash, a non-volatile non-writable part, e.g., ROM.

Storage 140 may be non-transitory storage. For example, storage 140 may store data in the presence of power such as a volatile memory device, e.g., a Random Access Memory (RAM). For example, storage 140 may store data in the presence of power as well as outside the presence of power such as a non-volatile memory device, e.g., Flash memory.

Device 110 may be connected to a database 112. Database 112 may be internal or external to device 110. For example, device 110 may store input images, segmentations, corrections, and so on. Database 112 may be shared by multiple devices 110, e.g., to facilitate multiple experts working in parallel, e.g., improving training data in parallel.

The device 110 may communicate internally, with other systems, with other devices, external storage, input devices, output devices, and/or one or more sensors over a computer network. The computer network may be an internet, an intranet, a LAN, a WLAN, etc. The computer network may be the Internet. The device 110 comprise a connection interface which is arranged to communicate within system 100 or outside of system 100 as needed. For example, the connection interface may comprise a connector, e.g., a wired connector, e.g., an Ethernet connector, an optical connector, etc., or a wireless connector, e.g., an antenna, e.g., a Wi-Fi, 4G or 5G antenna.

The communication interface 150 may be used to send or receive digital data, e.g., to receive an input image, to receive a correction, to send a segmentation image, a correction image, an uncertainty image, an error image, and so on.

The execution of device 110 may be implemented in a processor system. The device 110 may comprise functional units to implement aspects of embodiments. The functional units may be part of the processor system. For example, functional units shown herein may be wholly or partially implemented in computer instructions that are stored in a storage of the device and executable by the processor system.

The processor system may comprise one or more processor circuits, e.g., microprocessors, CPUs, GPUs, etc. Device 110 may comprise multiple processors. A processor circuit may be implemented in a distributed fashion, e.g., as multiple sub-processor circuits. For example, device 110 may use cloud computing. In an embodiment, device 110 is a locally integrated device. In an embodiment, device 110 is a distributed over multiple geographically different locations.

Typically, the image segmentation device 110 comprises a microprocessor which executes appropriate software stored at the device; for example, that software may have been downloaded and/or stored in a corresponding memory, e.g., a volatile memory such as RAM or a non-volatile memory such as Flash.

Instead of using software to implement a function, device 110 may, in whole or in part, be implemented in programmable logic, e.g., as field-programmable gate array (FPGA). The device may be implemented, in whole or in part, as a so-called application-specific integrated circuit (ASIC), e.g., an integrated circuit (IC) customized for their particular use. For example, the circuits may be implemented in CMOS, e.g., using a hardware description language such as Verilog, VHDL, etc. In particular, image segmentation device 110 may comprise circuits, e.g., for neural network processing, and/or arithmetic processing.

The execution of image segmentation devices and/or methods may be implemented in a processor circuit, examples of which are shown herein. Figures 2a-2c show functional units that may be functional units of the processor circuit. For example, the Figures may be used as a blueprint of a possible functional organization of the processor circuit. Other organizations than those shown in these Figures are possible.

The processor circuit is not shown separate from the units in those Figures. For example, the functional units may be wholly or partially implemented in computer instructions that are stored at device 100, e.g., in an electronic memory of device 100, and are executable by a microprocessor of device 100. In hybrid embodiments, functional units are implemented partially in hardware, e.g., as coprocessors, e.g., neural network coprocessors, and partially in software stored and executed on the device.

Figure 2a schematically shows an example of an embodiment of an image segmentation system 200. Image segmentation system 200 may for example be implemented in device such as device 110. For example, in an embodiment, a workstation or imaging apparatus comprises system 200.

System 200 is configured with an image segmentation model 210. The image segmentation model 210 is configured to an input image and to produce as output a segmentation image classifying parts of the input image. Figure 2a shows an input image 213 and a segmentation image 211.

The image segmentation model is advantageously a medical segmentation model; image segmentation model 213 could also be applied to different types of images, e.g., images of manufactured products, street images and the like.

The input image 213 may be obtained from a various medical imaging technologies. Input image 213 may be multi-dimensional image data, e.g., two-dimensional (2D), three-dimensional (3D) or four-dimensional (4D) images. Input image 213 may be acquired by various acquisition modalities such as, but not limited to, standard X-ray Imaging, Full-Field Digital Mammograms, Computed Tomography (CT), Magnetic Resonance Imaging (MRI), Ultrasound (US), Positron Emission Tomography (PET), Single Photon Emission Computed Tomography (SPECT), and Nuclear Medicine (NM). Image segmentation model 210 may be configured for one or more of these image modalities.

Image segmentation model 210 is at least partially trained, that is, image segmentation model 210 is indeed capable of generating a segmentation image 211. However, there is no need that that image segmentation model 210 is fully trained. As a result some errors in the segmentations of image segmentation model 210 is acceptable. Especially, errors that are due to imperfect training material. Embodiments may be applied to improve and/or acquire training data with fewer errors.

Image segmentation may comprise the identification of regions of interest (ROIs) in image data. Medical image segmentation may be used for example in computer-aided diagnosis. Image segmentation may, e.g., segment areas in an image, e.g., segmenting body organs, tissues, tumor detection and/or segmentation, and mass detection. For example, an image segmentation model may segment a tumor in an image of a patient's breast. Image segmentation may also be used to simulate physical properties in silico, or virtually positioning CAD-designed implants within a patient.

For example, in an embodiment, a medical image may be obtained, say an MRI image, the image segmentation model may be applied to the image to obtain a segmentation image. The segmentation image indicates a segmentation into one or more regions of interest, e.g., to isolate breast tissue and a tumor therein. A segmentation image may comprise or be associated with labels indicating the type of segmentation. The resulting segmentation may be used for diagnosis, planning potential treatments, and the like. The segmentation of medical images may help, e.g., in checking the growth of a tumor, controlling the dosage of medicine, and dosage of exposure to radiations.

For example, MRI Planning may involve aligning a box geometry in line with anatomical features. Using an image segmentation model, instant box planning is possible based on, e.g., a survey scan or previous scan.

Image segmentation model 210 is a machine learnable model, that can be trained and/or refined on training data, e.g., comprising multiple training items. A training item may comprise a sample image and a desired outcome, e.g., a segmentation that the model ought to produce.

An image segmentation model may be based on various artificially intelligent technologies. Segmentation techniques include classification and clustering methods, such as nearest neighbors models, support vector machines (SVM), and artificial neural networks (ANNs), in particular convolutional neural networks. Especially neural network based image segmentation show high promise in segmentation. Neural networks are improved by using a higher quality training set, though any machine learnable model that relies on training data is improved by improving the quality of the training data. The image segmentation model may comprise a convolutional neural network (CNN), e.g., a neural network comprising one or more convolutional layers. For example, the image segmentation model may comprise, a so-called u-net.

The segmentation may be for one particular element. For example, in an embodiment, an image segmentation model may be trained to segment in an image a tumor tissue only, e.g., in a mammograph. The segmentation may be for multiple elements. For example, in an embodiment, an image segmentation model may be trained to segment different kinds of tissues, organs or the like in an image. For example, this may be implemented by producing multiple images, e.g., one for each label, or by associating each pixel or voxel with a label vector indicating which label applies, or which a probability label vector, indicating the probabilities of various labels, etc. Segmentation for multiple labels may also be indicated with visual elements, such as a fill with a color or pattern, a boundary curve, etc.

In an embodiment, system 200 is configured to apply image segmentation model 210 to a set of input images thus obtaining a corresponding set of segmentation images. In Figure 2a, one input image 213 and one segmentation image 211 is shown. The set of segmentation images may be partially correct and partially faulty.

A set of corrected segmentation images is obtained for the set of input images. The corrections are obtained from expert judgement, likely a person skilled in the particular field of interest, e.g., a radiologist. Possibly expert judgement could be a different artificial intelligence (not shown) trained in some other way, e.g., obtained from a third party and which may be used to train image model 210. For example, the different model could be a previous generation image segmentation model, that has high quality segmentation, but for some reason is to be replaced by image model 210. We will assume herein that expert judgement is obtained from expert persons, but embodiments could be modified to rely on other types of expert judgement.

For example, system 200 may comprise an expert judgement interface 230. Expert judgement interface 230 may be configured to enable an expert to make corrections to a segmentation image, thus obtaining a corrected segmentation image. Figure 2a shows a corrected segmentation image 231 corresponding to input image 213 and segmentation image 211.

The corrected segmentation image may be used for improving image model 210, e.g., a training item may comprise an input image 211 and a corresponding corrected segmentation image. The training of image model 210 may be a complete retraining of a new image segmentation model, possibly with the addition of additional training data. The training of image model 210 may also be a retraining or refinement, e.g., using the existing image model 210 as a starting point for additional training iterations; possibly existing image model 210 is adapter by introducing noise, dropout, or the like.

Figure 2a shows an embodiment that includes an element configured for training the image segmentation model 210, i.e., first model trainer 241. For example, first model trainer 241 may be configured for a backpropagation algorithm. For example, first model trainer 241 may be configured to use an Adam optimizer.

In addition to the corrected segmentation images, a corresponding set of error images is obtained. Figure 2a shows one error image 232 corresponding to the corrections made in corrected segmentation 231. Error image 232 may be automatically derived from corrected segmentation 231, or vice versa. An error image may use the same format as an input image or as a segmentation image. For example, the error image may comprise a two-dimensional collection of gray values, or of label vectors or the like. In particular, values in the error image, e.g., pixel values may indicate the likeness of correction by an error. An error image may use simpler format though, e.g., a binary format, for example.

For example, an error image may be a difference image, e.g., indicating a difference between a segmentation image produced by model 210 and the corresponding corrected segmentation image. The error image may be obtained in other ways. For example, the error image may indicate where a segmentation was correct, e.g., where a boundary was corrected, e.g., where a point of the boundary was pulled or pushed, e.g., where a supporting point on a spline of the like was moved. In any case, the error image indicates a location where the segmentation image was corrected by the expert.

Once a set of error images has been obtained, a further image segmentation model 220 may be trained to predict an expected error image for an input image. Further image segmentation 220 model may use the same image segmentation technology as image segmentation model 210. For example, model 220 may also comprise a neural network, e.g., a CNN. Image segmentation model 220 may be smaller than image segmentation model 210. For example, image segmentation model 220 may have fewer layers, and/or fewer neurons. For example, image segmentation model 220 may have at most half the number of neurons as image segmentation model 210. This is possible, as the quality of the segmentation of model is allowed to be less high than those of model 210. This is not necessary though, and image segmentation model 220 may be as large or larger than model 210.

Further image segmentation model 220 may in part use the same trained parameters as image model 210. For example, image segmentation models 210 and 220 may be a different head on top of a common body.

Further image segmentation model 220 learns to predict where an expert may correct a segmentation image. This is advantageous and helps to overcome challenges in manual and semi-automatic labelling of training data.

Figure 2a shows an embodiment that includes an element configured for training the further image segmentation model 220, i.e., second model trainer 242. For example, second model trainer 242 may be configured for a backpropagation algorithm. For example, first model trainer 242 may be configured for the same training algorithm as trainer 241.

There are various ways to obtain corrections, e.g., using the expert judgement interface 230. For example, in an embodiment image segmentation model 210 may be applied to input image 213 to obtaining a corresponding segmentation image 211. The further image segmentation model may then be applied to obtain an expected error image 221 corresponding to input image 213 and segmentation image 213. For example, expert judgement interface 230 may be configured to display both the segmentation image 211 and the expected error image 221 to an expert. For example, the displaying may use a display, e.g., a monitor.

The expert may use a user interface to enter a correction. For example, he may use a pointing tool, such as a mouse, stylus, or the like, to identify a point on a segmentation boundary. For example, the point could be pushed or pulled by the expert. For example, the expert may use the pointing device to draw a new boundary of the segmentation. If the expert judgement is not human judgement, other interface may be more useful, e.g., a digital interface.

A corrected segmentation image for the segmentation image is thus obtained from the expert. The error image may be derived therefrom.

Embodiments have several advantages. For example, some of the issues that an embodiment can address include.

*Availability of experts:* Experts, e.g., radiologists have various roles and responsibilities to fulfil. Yet the expert plays a key role in obtaining good accuracy labels. Considering these parameters it is difficult and costly to obtain sufficient expert hours with a sufficiently high skill level for medical image annotation. Using a further image model according to an embodiment, expert time is used more productively by pointing out the uncertainties in the labelling process. Accordingly, image segmentations produced by model 210 will be of higher quality as the model generalizes a larger pool of expert annotations and/or a higher quality pool.

*Labor-intensive and Time-consuming*: In a normal clinical setting, a trained radiologist takes several minutes or more based on the complexity of the image to review or edit a medical image segmentation area and prepare the report. Segmentation Labelling of medical image through Semi-Automatic Methods takes approximately 5-10 minutes. Manual methods take even longer. As training image segmentation models uses large volumes of training data, the task of labelling becomes intensive and time-consuming. A dynamic approach including learning from real-world uncertainty knowledge graphs derived from various human annotators shortens review time.

*Intra and Inter-Annotator Variance*: A further issue is the variance that is found in the manual annotations by one or multiple annotators. For example, due to a different skill level, the interpretation of same medical image may differ between different radiologists. Semi-automatic methods do better in comparison with manual methods, but they too are prone to the variance. This issue leads to poorer quality of labels for the image segmentation model. An image segmentation model that is trained explicitly on the areas in an image where corrections are actually made, will focus expert attention on a part of the image where other experts on similar images made corrections, and hence, the current expert may consider correction as well. Without this information the expert might not have considered the correction. Accordingly, the variation is reduced.

*Availability of Representative Data*: One of the challenges for training machine learnable models, e.g., neural networks is the limited availability of medical imaging data for training. Medical Data availability is limited due to various factors like regulatory, cost, etc. Also, within the data available, the data may not be representative of all the variations. A machine learnable model may fail in this aspect if the unseen data has variations with respect to training data. The proposed invention addresses this crucial aspect by providing information on the failure cases by identifying likely human label corrections.

Segmentation tasks like labelling, classification, landmark annotation can benefit from embodiments, etc.

Figure 2a shows a configuration that includes an element configured for training the image segmentation model 210, i.e., first model trainer 241, and an element configured for training the further image segmentation model 220, i.e., second model trainer 242.

Neither first model trainer 241 nor second model trainer 242 is necessary, and either or both could be omitted. For example, in an embodiment, system 200 may not be configured for training or retraining model 210, e.g., when only a further image segmentation model 220 is desired. Furthermore, training may be complete, or may be done on a different device or system. For example, in an embodiment, system 200 may not be configured for training or retraining model 220, e.g., a pretrained model may be obtained from another source.

For example, an embodiment may use an already trained further image segmentation model. Such a system may be configured, say to apply the further image segmentation model to obtain expected error image 221 and display the expected error image 232 to an expert to guide him/her in correcting the segmentation image. Such correct images may then be used to refining and/or retrain the image segmentation model 210. The error image may be displayed superimposed over input image 213, or a copy of input image 213, as is convenient.

Having an accurate further image segmentation model 220 is desirable, but the penalty of having an occasional faulty prediction is less severe than for model 210, so that training of model 220 may be aborted sooner than for model 210.

Figure 2a shows an optional uncertainty image 212 generated for input image 213. An uncertainty image shows the confidence of image segmentation model 210 for different parts of the segmentation image. Obtaining uncertainty images does not require training on corrections of experts, e.g., on error images. Uncertainty images are relatively easy to obtain, which is an advantage, but they have various downsides. Uncertainty images could be shown to experts, but the inventors found that they are more advantageously used as input, possibly an additional input, to the further image segmentation model 220. Uncertainty images are further discussed herein.

Further image segmentation model 220 may be configured to receive as input uncertainty image 212, but not input image 213

Further image segmentation model 220 may be configured to receive input image 213 as input, but no uncertainty image 212 is used. This option may be suitable if a relatively powerful image segmentation model is trained for the further image segmentation model 220.

Further image segmentation model 220 may be configured to receive both input uncertainty image 212, and input image 213.

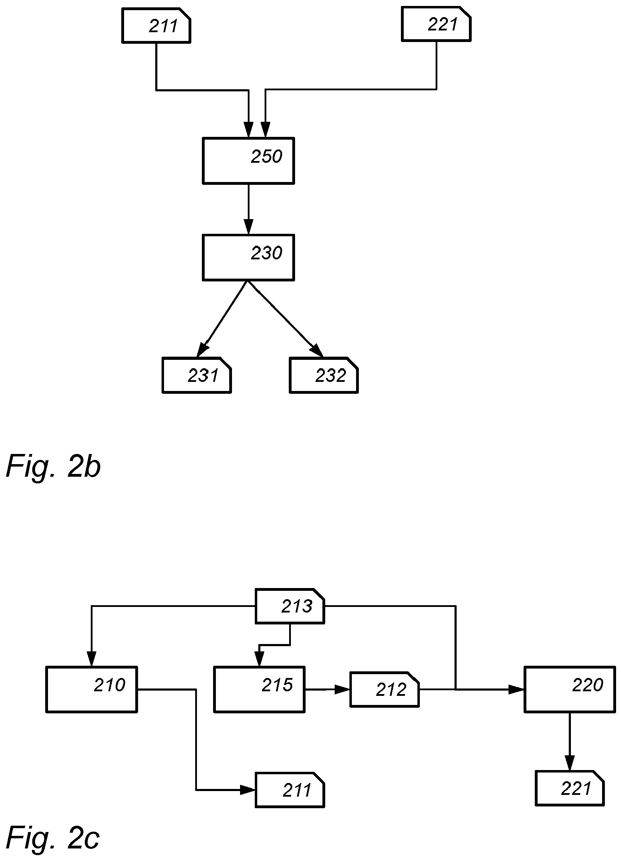

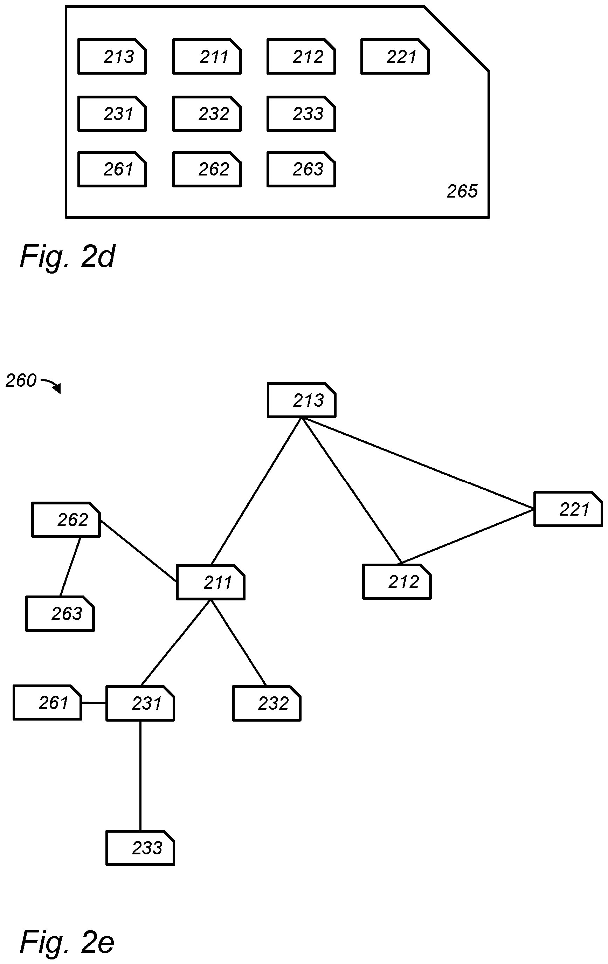

Figure 2b schematically shows an example of an embodiment of an image segmentation system. Figure 2b shows an optional detail of expert correction. Figure 2b, e.g., the expert selection unit 250 may be incorporated in system 200.

The expert judgement may be obtained from at least one expert in a pool of experts. Which expert to use may make a difference. For some corrections a higher skill level may be needed than for other corrections. Figure 2b shows an expert selection unit 250. Expert selection unit 250 is configured to select an expert from the pool of experts to obtain a corrected segmentation images. Which expert to select may depend on a level of uncertainty in the uncertainty image corresponding to the segmentation image. For example, if expected error image 221 shows that larger parts of the image might need correction, then an expert with a higher skill level may be needed for the correction.

For example, unit 250 may comprise a list of experts and a corresponding skill set, e.g., a segmentation skill level. Segmentation skill level may be general or specific for a type of image. Unit 250 may match a segmentation to an expert based on an uncertainty level in the image, but may also use other factors. For example, an expert in mammography may preferably handle corrections in this area.

The level of uncertainty may be derived from expected error image 221, but the level of uncertainty may also be derived from additional or alternative sources, e.g., input image 211 and an uncertainty image 212, discussed herein. For example, level of uncertainty may be the area of the segmentation image that may need correction. The level of uncertainty may be an integral over uncertainty in the segmentation image, e.g., summing uncertainty over the image.

In an embodiment, if the uncertainty in an image is low enough no correction may be required at all. For example, system 200 may be configured to determine for a segmentation image an uncertainty level indicating an overall expected error. The expected error may be computed from the associated expected error image and/or from an associated uncertainty image. If the uncertainty level is below a threshold indicating a low expectation of error, then the correction step may be skipped. Input image 213 and segmentation image 211 may still be added to training data.

The accepted uncertainty level is the threshold value to filter the high and low uncertain samples. The value of the threshold value can be determined empirically, e.g., by evaluating a sample of rejected and accepted images. The value of the threshold value may also be determined by requiring that some fixed percentage of images is verified, e.g., 50%, 10%, etc.

In an embodiment, system 200 is configured to order, e.g., rank the set of segmentation images and get labels first for prioritized samples; For example, samples that are expected to need correction. This helps to increase performance of the segmentation model quicker.

Figure 2c schematically shows an example of an embodiment of an image segmentation system. Figure 2c shows an optional uncertainty model. For example, the uncertainty model may be incorporated in system 200. Figure 2c shows an associated uncertainty model 215 that is configured to generate an uncertainty image 212 for a given input image 213.

The associated uncertainty model is configured to produce an uncertainty image for an input image indicating a confidence of the image segmentation model for different parts of the segmentation image. The uncertainty model may be applied to the set of training data thus obtaining a set of uncertainty images corresponding to the set of segmentation images, the further image segmentation model taking as input at least the corresponding uncertainty image. For example, an uncertainty image may estimate variance in multiple potential segmentations for pixels of an input image.

There are various ways in which an associated uncertainty model may be obtained. For example, the uncertainty model may be obtained together with the image segmentation model. For example, as byproduct of training the uncertainty model may be created. For example, the uncertainty model may be different head on a body shared with the image segmentation model.

Various ways to create uncertainty models are known, and these can be applied in the context of an embodiment.

For example, associated uncertainty model 215 may be configured to estimate variance in multiple potential segmentations for pixels of an input image. For example, multiple potential segmentations may be obtained in addition to segmentation 211. For example, multiple potential segmentations may be obtained from one or more of: probabilistic segmentations for multiple labels, an ensemble of segmentation models, multiple heads, and/or iterated Monte-Carlo dropout. A variance computed over the multiple segmentation images, e.g., computed per pixel may be taken as an uncertainty image.

Examples of known uncertainty estimators can be found in the paper "Estimating uncertainty in deep learning for reporting confidence to clinicians in medical image segmentation and diseases detection", by Biraja Ghoshal. The paper gives various option, in particular, section 4.1 provides a worked example for a U-net. Such an uncertainty model could be used for model 215.

In addition to uncertainty images, an uncertainty score may be calculated based on the uncertainty image obtained. For example, the uncertainty score may be a sum, a maximum, an integral computed over the uncertainty image. Like total expected error obtained from an expected error image, the uncertainty score may be compared to a threshold value to decide if review is needed, and/or whether review by a highly skilled or lower skilled reviewer is needed. The threshold can be defined by the annotators as based on acceptance error, or it can be manually calculated based on the model performance on validation data set, etc.

In an embodiment, a segmentation may be obtained from image segmentation model 210, e.g., based on a pre-trained model to predict a segmentation. Uncertainty model 215 may be applied to obtain an uncertainty image. The uncertainty image may be displayed to experts when correcting a segmentation. This may be used, e.g., before model 220 is trained. Once model 220 is trained, an expected error image may be displayed instead of, or in addition to, the uncertainty image.

Uncertainty images reflect in part the areas where the training data has inter-or intra-expert variability. Even if the same image is segmented by the same expert, then he/she will not put the segmentation boundaries at exactly the same places. The variation may be worse if a pool of experts is used, used and different images are considered. As a result image model 210 will naturally be uncertain about segment boundaries-reflecting the uncertainty present in the training data. However, in practice such boundaries are often not corrected, as the boundary appears to be placed sufficiently accurate. This causes a distracting uncertainty halo around objects. Model 220 trained on actual corrections is less affected by this issue.

In one example uncertainty algorithm, a base image segmentation model is obtained. In an embodiment, a dropout layer is added to model 210 after a convolution layer, or each convolutional layer, and/or as additional layers at the end of the network architecture. Bayesian uncertainty detection may use the dropout layers to detect uncertainty. Variational Inference method may add two fully connected layers similar to the final layer of the network. After modifying the existing network into an uncertainty detection network, the model may be retrained with the training data.

During prediction, model 210 may be run for a defined number of Monte Carlo iterations. The mean and variance may be captured for each sample. The uncertainty of the sample may be approximated as the calculated variance. Various uncertainty models may be adopted from the state of the art.

Embodiments can produce a large quantity of related data. In an embodiment, a data structure comprises at least an input image 213, and a corrected segmentation image 231, however much more data may be maintained. Figure 2d schematically shows an example of an embodiment of a data structure 265. Shown in Figure 2d, the data structure 265 is associated with input image 213, segmentation image 211, uncertainty image 212, expected error image 221, corrected segmentation image 231, error image 232, e.g., as discussed herein. An embodiment may use yet further data though. For example, the same input image may be segmented by different various of model 210 leading to multiple segmentation images; the same segmentation image may be corrected by multiple experts leading to multiple corrected segmentation images (shown is one such further corrected segmentation image 233). Beyond that many types of addition data may be kept, e.g., who corrected an image, when did this happen, what was his or her skill level, correction of labels in addition or instead of correction of areas in a segmentation, and so on. Shown are three additional data 261-263, there may be fewer or more additional data than 3. The data structure may be a file, a database, a list of pointers or the like.

The data that is stored is not necessarily constant, and can dynamically change. The inventors found that an advantageous data structure to use is a knowledge graph. Figure 2e schematically shows an example of an embodiment of a knowledge graph 260. Knowledge graph 260 contains the same information as data structure 265, but is can easily be extended or modified as more information is obtained in some way related to image 213.

Shown in Figure 2e, the knowledge graph 260 comprises input image 213. Input image 213 is associated with, e.g., linked with, segmentation image 211, uncertainty image 212, and expected error image 221. The latter three images are all obtained from the input image by applying one or more of model 210, uncertainty model 215 and further model 220.

Segmentation image 211 is associated with corrected segmentation image 231, and error image 232. Further corrected segmentation image 233 is shown associated with corrected segmentation image 231, e.g., to reflect this is a review of a reviewer; further corrected segmentation image 233 could be associated with segmentation image 211 instead, or as well, to reflect that it is also a segmentation of this image. Further data 261-263 is linked to images wherever that is appropriate.

The knowledge graph thus provides a dynamic, flexible data structure in which varying amounts of data can be efficiently stored.

For example, in an embodiment multiple radiologists and/or human annotators may feed a continuous learning system for segmenting, e.g., labelling, images, but also to train or refine the models 210 and 220. Model 210 may integrate the derived knowledge from the annotator through the knowledge graph. The knowledge graph may track annotator actions during the correction of uncertainty regions. These actions may then be integrated into the existing trained model to reduce uncertain in the segmentations. The updated model 210/220 may then be integrated into system 200 to further optimize human intervention.

For example, model 210 may segment an input image 213 obtaining a segmentation 211 and an (optional) uncertainty image 212. If uncertainty is sufficiently low, the segmentation 211 may be immediately sent to a labeled image pool of labeled images.

Uncertain segmentation may be sent to expert annotators. The experts correct the segmentation. The provenance may be stored in a knowledge graph. Corrected segmentation may be stored in the labeled image pool.

Once a sufficient number of corrected segmentations are obtained, the further model 220 may be trained to produce an expected error image. Input image 213 and/or uncertainty image 212, and possibly other information, e.g., as stored in the knowledge graph, may be used to train further model 220. We refer to the uncertainty model, e.g., model 215 as a weak uncertainty detector and model 220 as a strong uncertainty detector. The strong uncertainty detector can detect uncertainty more precisely, e.g., to reduce human intervention. Accordingly, the strong uncertainty detector may be used to guide experts, rather than the uncertainty images; though uncertainty images may still be used as an input for model 220. Once the labeled image pool is large enough, models 210 and/or 220 may be refined, retrained, or replaced by a newly trained model. Once the labeled image pool is large enough, a production-grade image segmentation model may be trained, e.g., having more layers, and/or neurons than the model 210 used to support labelling.

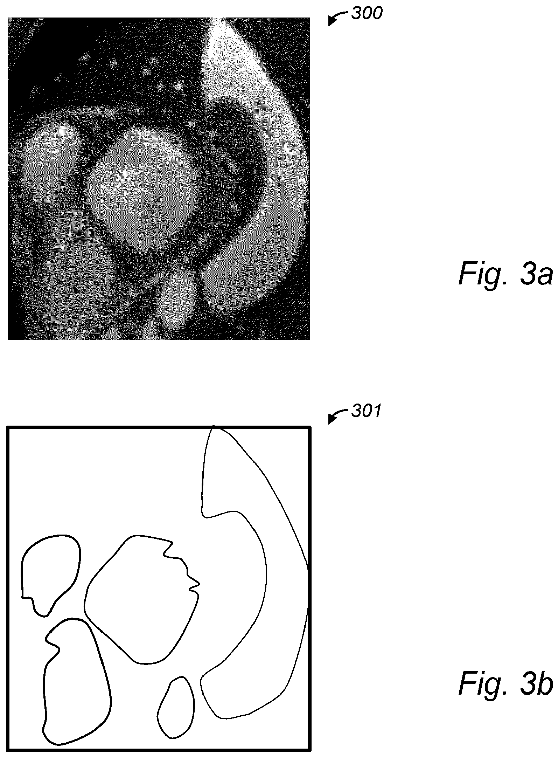

Figure 3a shows an example of an embodiment of a medical image 300. Medical image 300 is a reproduction of an actual medical image. For example, image 300 could an input image for an image segmentation model, e.g., such as input image 213.

Figure 3b schematically shows an example of an embodiment of a medical image 301. Medical image 301 is a schematic representation of medical image 300. Medical image 301 has been drafted by hand following medical image 300. The images shown in Figures 3b-3g are hand-drawn schematic Figures, although they correspond schematically to actual two-dimensional images taken from a prototype of an embodiment following Figure 2a. To improve clarity and avoid the use of colors the images have been replaced herein by hand drawn representations.

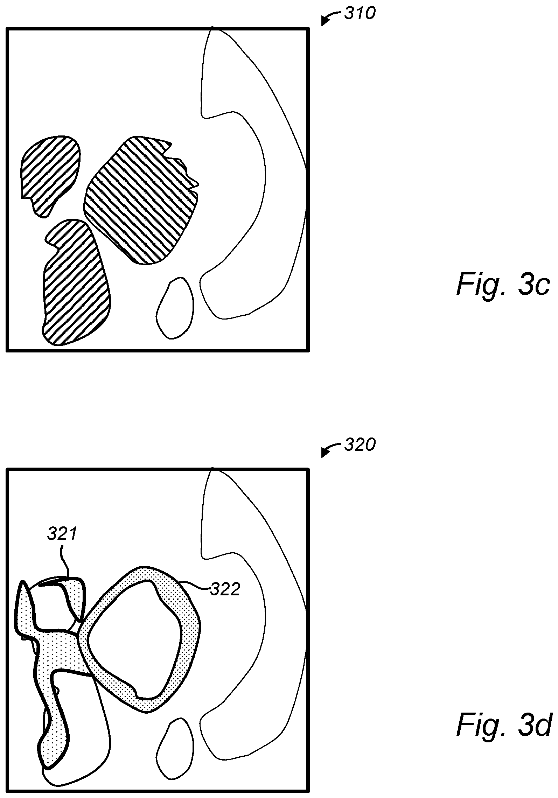

Figure 3c schematically shows an example of an embodiment of a segmentation image 310. For example, segmentation image 310 may be produced by an image segmentation mode, e.g., such as a segmentation image 211 produced by model 210. Visible in segmentation image 310 are two objects showing a different pattern fill. The pattern fill visualizes that the image segmentation model 210 identified two objects in the image. In an embodiment, instead of a pattern fill, the image could use color to indicate a segmentation, or use a boundary that is moving or flashing or colored or any other visual indication of the segmentation produced.

Figure 3d schematically shows an example of an embodiment of an uncertainty image 320.

Uncertainty image 320 is produced by an uncertainty algorithm. For example, the uncertainty image may reflect that a varying models and/or using variant training data would produce the same segmentation. An uncertainty image thus gives information about the likelihood that a segmentation is correct.

In Figure 3d, areas with an uncertainty exceeding a threshold have been indicated with a pattern. Area 322 surround a segmented object with a halo of uncertainty. Area 321 shows that large parts of the underlying object are indicated as uncertain.

An expert tasked with correcting image 310 may be presented, say, input images 300 (301), segmentation 310 and uncertainty image 320. However, the utility of uncertainty image 320 is limited. For example, the uncertainty halo 322 is likely due to variability in the training data, that does not correspond with a shortage of knowledge, but with the fact that the perfect location of such a boundary is not clear. As image 320 shows large part of the image as uncertain, expert focus is not guided where it is most needed.

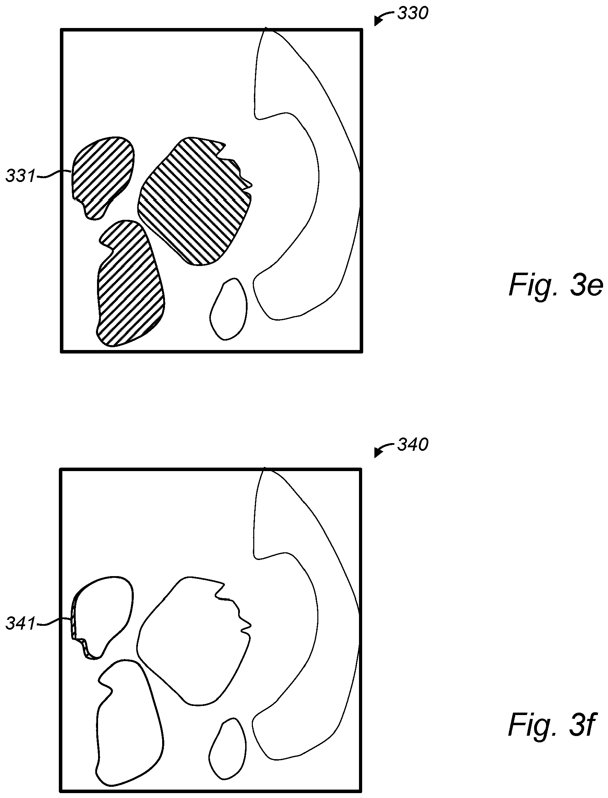

Figure 3e schematically shows an example of an embodiment of a corrected segmentation image 330. The expert concluded that the segmentation shown in Figure 3c is essentially correct, except for part of the boundary indicated at 331. The correction is entered by the expert through a suitable user interface, probably a graphical user interface with a corresponding pointing input device.

Figure 3f schematically shows an example of an embodiment of an error image 340. Error image 340 shows the difference between images 330 and 301. Note that only a slim strip at the edge, indicated at 341, is indicated as corrected, e.g., as in-error.

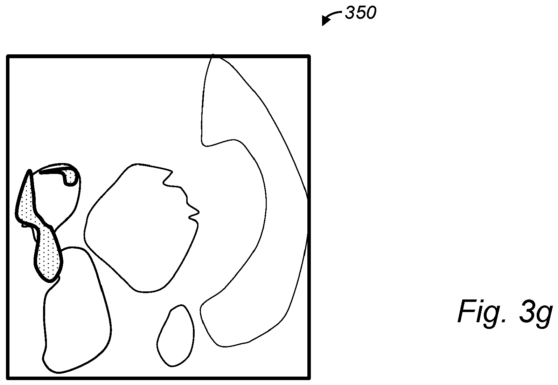

Figure 3g schematically shows an example of an embodiment of an expected error image 350. The Figure shows the output of a trained further image segmentation model. The model indicates the locations where corrections are expected.

In an embodiment, the expert might be shown, say, input images 300 (301), segmentation 310 and expected error image 350. Image 350 has much higher utility for the expert. He/she can now focus on the parts that are likely to be erroneous. A reduced input time for the image is thus expected.

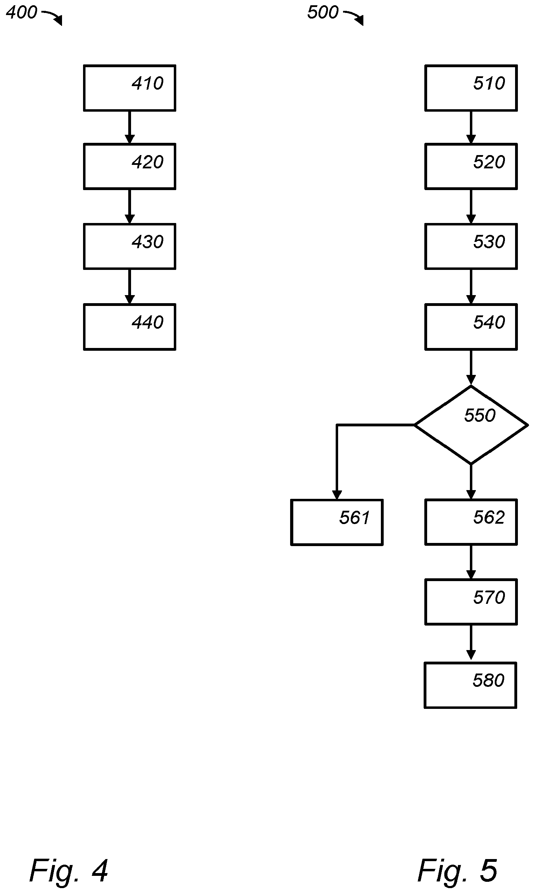

Figure 4 schematically shows an example of an embodiment of an image segmentation method 400. Image segmentation method 400 may be implemented using at least one computer processor. Method 400 may comprise:
- obtaining (410) an image segmentation model, the image segmentation model being at least partially trained,
- applying (420) the image segmentation model to a set of input images thus obtaining a corresponding set of segmentation images,
- obtaining (430) a set of corrected segmentation images correcting the set of segmentation images from expert judgement and a corresponding set of error images, an error image indicating a location where a segmentation image is corrected,
- training (440) a further image segmentation model predicting an expected error image for an input image.

Figure 5 schematically shows an example of an embodiment of an image segmentation method 500. Method 500 may comprise:
510: collecting a small training set
520: training a simple expected error model
530: gather a large set of unlabeled data
540: make segmentations on the unlabeled data using an image segmentation model
550: determine if uncertainty in a labelling exceed a threshold according to the expected error model
561: if uncertainty is low, add the labeled image to a labeled image pool
562: label the most uncertain images using expert judgement
570: add the expert labeled image to the labeled image pool
580: retrain the image segmentation model and/or the expected error.

Method 500 could be extended or changed in various ways. For example, an uncertainty model could be added, to be used as input for the expected error model.

Many different ways of executing the method are possible, as will be apparent to a person skilled in the art. For example, the order of the steps can be performed in the shown order, but the order of the steps can be varied or some steps may be executed in parallel. Moreover, in between steps other method steps may be inserted. The inserted steps may represent refinements of the method such as described herein, or may be unrelated to the method. For example, some steps may be executed, at least partially, in parallel. Moreover, a given step may not have finished completely before a next step is started.

Embodiments of the method may be executed using software, which comprises instructions for causing a processor system to perform method 400 or 500. Software may only include those steps taken by a particular sub-entity of the system. The software may be stored in a suitable storage medium, such as a hard disk, a floppy, a memory, an optical disc, etc. The software may be sent as a signal along a wire, or wireless, or using a data network, e.g., the Internet. The software may be made available for download and/or for remote usage on a server. Embodiments of the method may be executed using a bitstream arranged to configure programmable logic, e.g., a field-programmable gate array (FPGA), to perform the method.

It will be appreciated that the presently disclosed subject matter also extends to computer programs, particularly computer programs on or in a carrier, adapted for putting the presently disclosed subject matter into practice. The program may be in the form of source code, object code, a code intermediate source, and object code such as partially compiled form, or in any other form suitable for use in the implementation of an embodiment of the method. An embodiment relating to a computer program product comprises computer executable instructions corresponding to each of the processing steps of at least one of the methods set forth. These instructions may be subdivided into subroutines and/or be stored in one or more files that may be linked statically or dynamically. Another embodiment relating to a computer program product comprises computer executable instructions corresponding to each of the devices, units and/or parts of at least one of the systems and/or products set forth.

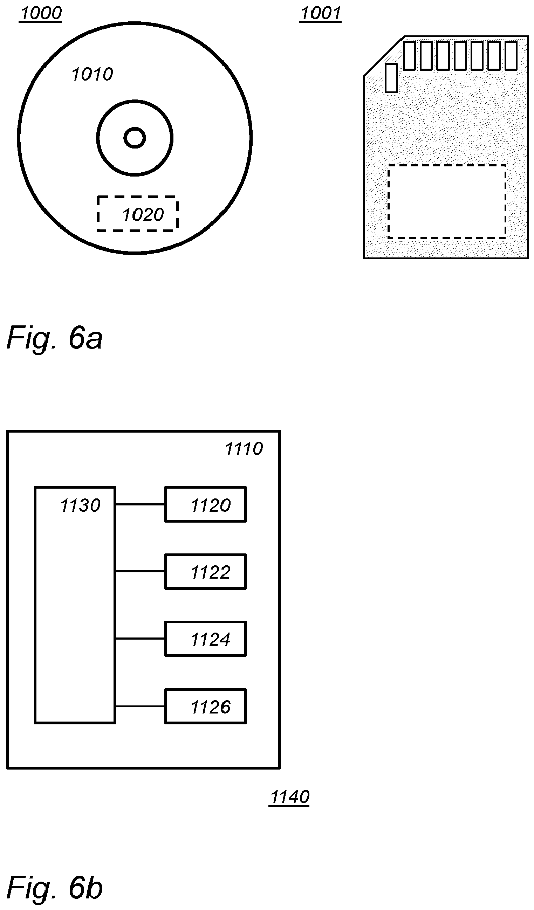

Figure 6a shows a computer readable medium 1000 having a writable part 1010, and a computer readable medium 1001 also having a writable part. Computer readable medium 1000 is shown in the form of an optically readable medium. Computer readable medium 1001 is shown in the form of an electronic memory, in this case a memory card. Computer readable medium 1000 and 1001 may store data 1020 wherein the data may indicate instructions, which when executed by a processor system, cause a processor system to perform an image segmentation method, according to an embodiment. The computer program 1020 may be embodied on the computer readable medium 1000 as physical marks or by magnetization of the computer readable medium 1000. However, any other suitable embodiment is conceivable as well. Furthermore, it will be appreciated that, although the computer readable medium 1000 is shown here as an optical disc, the computer readable medium 1000 may be any suitable computer readable medium, such as a hard disk, solid state memory, flash memory, etc., and may be non-recordable or recordable. The computer program 1020 comprises instructions for causing a processor system to perform said image segmentation method.

Figure 6b shows in a schematic representation of a processor system 1140 according to an embodiment of an image segmentation device. The processor system comprises one or more integrated circuits 1110. The architecture of the one or more integrated circuits 1110 is schematically shown in Figure 6b. Circuit 1110 comprises a processing unit 1120, e.g., a CPU, for running computer program components to execute a method according to an embodiment and/or implement its modules or units. Circuit 1110 comprises a memory 1122 for storing programming code, data, etc. Part of memory 1122 may be read-only. Circuit 1110 may comprise a communication element 1126, e.g., an antenna, connectors or both, and the like. Circuit 1110 may comprise a dedicated integrated circuit 1124 for performing part or all of the processing defined in the method. Processor 1120, memory 1122, dedicated IC 1124 and communication element 1126 may be connected to each other via an interconnect 1130, say a bus. The processor system 1110 may be arranged for contact and/or contact-less communication, using an antenna and/or connectors, respectively.

For example, in an embodiment, processor system 1140, e.g., the image segmentation device may comprise a processor circuit and a memory circuit, the processor being arranged to execute software stored in the memory circuit. For example, the processor circuit may be an Intel Core i7 processor, ARM Cortex-R8, etc. The memory circuit may be an ROM circuit, or a non-volatile memory, e.g., a flash memory. The memory circuit may be a volatile memory, e.g., an SRAM memory. In the latter case, the device may comprise a non-volatile software interface, e.g., a hard drive, a network interface, etc., arranged for providing the software.

While device 1110 is shown as including one of each described component, the various components may be duplicated in various embodiments. For example, the processor may include multiple microprocessors that are configured to independently execute the methods described herein or are configured to perform steps or subroutines of the methods described herein such that the multiple processors cooperate to achieve the functionality described herein. Further, where the device 1110 is implemented in a cloud computing system, the various hardware components may belong to separate physical systems. For example, the processor may include a first processor in a first server and a second processor in a second server.

It should be noted that the above-mentioned embodiments illustrate rather than limit the presently disclosed subject matter, and that those skilled in the art will be able to design many alternative embodiments.

In the claims, any reference signs placed between parentheses shall not be construed as limiting the claim. Use of the verb 'comprise' and its conjugations does not exclude the presence of elements or steps other than those stated in a claim. The article 'a' or 'an' preceding an element does not exclude the presence of a plurality of such elements. Expressions such as "at least one of' when preceding a list of elements represent a selection of all or of any subset of elements from the list. For example, the expression, "at least one of A, B, and C" should be understood as including only A, only B, only C, both A and B, both A and C, both B and C, or all of A, B, and C. The presently disclosed subject matter may be implemented by hardware comprising several distinct elements, and by a suitably programmed computer. In the device claim enumerating several parts, several of these parts may be embodied by one and the same item of hardware. The mere fact that certain measures are recited in mutually different dependent claims does not indicate that a combination of these measures cannot be used to advantage.

In the claims references in parentheses refer to reference signs in drawings of exemplifying embodiments or to formulas of embodiments, thus increasing the intelligibility of the claim. These references shall not be construed as limiting the claim.

## Claims

1. A method (400) for a machine learnable image segmentation model, an image segmentation model being configured to receive an input image and to produce as output a segmentation image classifying parts of the input image, the method comprising
- obtaining (410) an image segmentation model, the image segmentation model being at least partially trained,
- applying (420) the image segmentation model to a set of input images thus obtaining a corresponding set of segmentation images,
- obtaining (430) a set of corrected segmentation images correcting the set of segmentation images from expert judgement and a corresponding set of error images, an error image indicating a location where a segmentation image is corrected,
- training (440) a further image segmentation model predicting an expected error image for an input image.

2. A method as in Claim 1, wherein the image segmentation model is a medical segmentation model.

3. A method as in any one of the preceding claims, comprising
- applying the image segmentation model to an input image obtaining a corresponding segmentation image,
- applying the further image segmentation model to obtain an expected error image for an input image,
- displaying both the segmentation image and the expected error image to an expert,
- obtaining a corrected segmentation image for the segmentation image from the expert.

4. A method as in any one of the preceding claims, comprising capturing the error image and/or corrected segmentation image in a knowledge graph.

5. A method as in any one of the preceding claims, comprising training the image segmentation model on the expert corrected segmentation images.

6. A method as in any one of the preceding claims, wherein an associated uncertainty model is associated with the image segmentation model, the associated uncertainty model being configured to produce an uncertainty image for an input image indicating a confidence of the image segmentation model for different parts of the segmentation image, the method comprising applying the uncertainty model obtaining a set of uncertainty images corresponding to the set of segmentation images, the further image segmentation model taking as input at least the corresponding uncertainty image.

7. A method as in any one of the preceding claims, wherein the associated uncertainty model comprises estimating variance in multiple potential segmentations for pixels of an input image.

8. A method as in Claim 7, wherein the multiple potential segmentations may be obtained from one or more of:
- probabilistic segmentations for multiple labels,
- an ensemble of segmentation models,
- multiple heads, and/or
- iterated Monte-Carlo dropout.

9. A method as in any one of the claims 6-8, wherein the expert judgement is obtained from at least one expert in a pool of experts, the method comprising
- selecting an expert from the pool of experts to obtain a corrected segmentation images depending on a level of uncertainty in the uncertainty image corresponding to the segmentation image.

10. A method as in any one of the claims 6-9, comprising
- determining for a segmentation image an uncertainty level indicating an overall expected error from an associated expected error image and/or from an associated uncertainty image,
- only obtain the corrected segmentation image if the uncertainty level is above a threshold indicating a high expectation of error.

11. A method for a machine learnable image segmentation model, an image segmentation model being configured to receive an input image and to produce as output a segmentation image classifying parts of the input image, the method comprising
- obtaining an image segmentation model, the image segmentation model being at least partially trained,
- applying the image segmentation model to an input image thus obtaining a corresponding segmentation image,
- applying a further image segmentation model to obtain an expected error image for the input image, the further image segmentation model having been trained to predict an expected error image for an input image,
- displaying both the segmentation image and the expected error image to an expert, and
- obtaining a corrected segmentation image for the segmentation image from the expert.

12. A method for a machine learnable image segmentation model as in Claim 11, comprising
- training the image segmentation model on the expert corrected segmentation image.

13. A device for a machine learnable image segmentation model, an image segmentation model being configured to receive an input image and to produce as output a segmentation image classifying parts of the input image, the device comprising a communication interface configured for obtaining one or more input images, and a processor system configured for the method of any one of claims 1-12.

14. A transitory or non-transitory computer readable medium comprising
- computer instructions configured to, when the computer instructions are executed on a computer, to execute the method according to any one of claims 1-12, and/or
- parameters representing a further image segmentation model predicting an expected error image for an input image, trained according to Claim 1, and/or
- parameters representing an image segmentation model configured to produce a segmentation image, trained according to Claim 5 and/or Claim 12.
